# EUROPÄISCHE PATENTSCHRIFT

(11) **EP 3 125 978 B1**
(45) Veröffentlichungstag und Bekanntmachung des Hinweises auf die Patenterteilung: **24.01.2018**
(21) Anmeldenummer: 15708552.3
(22) Anmeldetag: 10.03.2015
(51) Int. Cl.: A61M 15/00

(54) **PHARMAZEUTISCHER SPENDER MIT EINER ERFASSUNGSEINRICHTUNG**
PHARMACEUTICAL DISPENSER WITH A DETECTION DEVICE
DISTRIBUTEUR DE PRODUITS PHARMACEUTIQUES ÉQUIPÉ D'UN MOYEN DE DÉTECTION

(30) Priorität: 02.04.2014 DE 102014206350
(43) Veröffentlichungstag der Anmeldung: 08.02.2017
(73) Patentinhaber: Aptar Radolfzell GmbH, 78315 Radolfzell (DE)
(72) Erfinder: KÖRNER, Joachim, 88690 Uhldingen-Mühlhofen (DE)
(74) Vertreter: Patentanwaltskanzlei Cartagena
(86) Internationale Anmeldenummer: PCT/EP2015/054981
(87) Internationale Veröffentlichungsnummer: WO 2015/150029

(56) Entgegenhaltungen:
- EP-A1- 2 397 178
- GB-A- 2 470 188
- US-A1- 2005 028 815

## Beschreibung

Die Erfindung betrifft einen pharmazeutischen Spender mit einem Gehäuse, einem Mediumspeicher, einer Austragöffnung sowie einen Verbindungskanal, der den Mediumspeicher mit der Austragöffnung verbindet. Ein gattungsgemäßer Spender weist weiterhin eine Erfassungseinrichtung auf, die im Bereich des Verbindungskanals angeordnet ist und zur Erfassung des im Verbindungskanal strömenden Mediums ausgebildet ist.

Gattungsgemäße Spender werden zum Austrag pharmazeutischer Medien, insbesondere pharmazeutischer Flüssigkeiten, genutzt. Insbesondere betrifft die Erfindung solche gattungsgemäßen Spender, die als Inhalatoren ausgebildet sind und der Erzeugung eines vernebelten Mediumstroms zum Zwecke der Inhalation dienen.

Bei gattungsgemäßen Spendern ist eine Erfassungseinrichtung vorgesehen, die in der Lage ist, in Reaktion auf einen Austragvorgang diesen zu erkennen und elektronisch zu verarbeiten bzw. als Signal zur elektronischen Verarbeitung an eine Verarbeitungseinrichtung weiterzugeben. Diese Verarbeitung kann insbesondere darauf abzielen, die erfolgten Austragvorgänge zu zählen, um abschätzen zu können, wie viele Austragvorgänge mit dem Spender bzw. dem im Spender eingesetzten Mediumspeicher noch möglich sind. Diese Information ist für Patienten von großer Wichtigkeit, um abschätzen zu können, ob sie mit der verbleibenden Menge pharmazeutischen Mediums auskommen oder einen neuen Spender bzw. einen neuen Mediumspeicher für den Spender benötigen.

Aus dem Stand der Technik ist es insbesondere bekannt, die manuelle Betätigung des Spenders selbst zu erfassen und zu zählen. Es hat sich jedoch in der Vergangenheit gezeigt, dass eine solche Erfassung nachteilig sein kann, da es hierbei sowohl zu ungezählten Austragvorgängen als auch zu gezählten Betätigungsvorgängen, die keinen Austrag verursacht haben, kommt.

Es ist daher bereits vorgeschlagen worden, nicht die Verlagerung mechanischer Teile des Spenders zu erfassen, welche im Zuge eines Austragvorgangs gegeneinander verlagert werden, sondern stattdessen eine physikalische Größe zu erfassen, die unmittelbar im Zusammenhang mit dem ausströmenden Medium steht. Aus der nachveröffentlichten DE 10 2013 214 601 B3 ist ein solcher Spender bekannt, bei dem im Bereich des Verbindungskanals eine Druckkammer vorgesehen, die abschnittsweise durch eine verformbare Membran begrenzt wird. Wenn das Medium durch den Verbindungskanal strömt, erhöht es hier den Druck und lenkt die Membran somit aus. Diese Auslenkung wird zur Betätigung eines Tasters genutzt, dessen Betätigung wiederum vom Zählwerk gezählt wird. Ein pharmazeutischer Spender mit den Merkmalen des Oberbegriffs des Anspruchs 1 ist aus der GB 2470188 bekannt.

### AUFGABE UND LÖSUNG

Aufgabe der Erfindung ist es, einen gattungsgemäßen Spender dahingehend weiterzubilden, dass dieser besonders zuverlässig eine Auswertung der Austragvorgänge gestattet. Diese Aufgabe wird mit einem pharmazeutischen Spender gemäß Anspruch 1 gelöst.

Erfindungsgemäß ist hierfür vorgesehen, dass der Verbindungskanal mit einer Druckkammer kommunizierend verbunden ist. Die Erfassungseinrichtung umfasst eine Membran, die diese Druckkammer zumindest abschnittsweise begrenzt und durch einen Druckanstieg in der Druckkammer auslenkbar ist. Die erfindungsgemäß vorgesehene Membran umfasst einen elektrisch leitfähigen Verbindungsabschnitt, der so in die Membran integriert ist, dass er bei einer Auslenkung der Membran gemeinsam mit dieser ausgelenkt wird.

Die Erfassungseinrichtung ist zum Zusammenwirken mit diesem Verbindungsabschnitt ausgebildet und verfügt hierfür über mindestens eine elektrische Gegenkontaktfläche, gegenüber der der membranseitige Verbindungsabschnitt durch Druckbeaufschlagung der Membran verlagerbar ist. Die Gegenkontaktfläche und der Verbindungsabschnitt an der Membran sind zueinander so angeordnet, dass durch die druckbedingte Verformung der Membran ein Kontakt zwischen dem leitfähigen Verbindungsabschnitt einer Membran und der Gegenkontaktfläche geschaffen oder getrennt werden kann.

Bei einem erfindungsgemäßen pharmazeutischen Spender ist somit vorgesehen, dass im Bereich des Verbindungskanals, durch den das Medium in Richtung der Austragöffnung strömt, eine Druckkammer vorgesehen ist, die entweder abgezweigt von einem Hauptkanal mit diesem kommunizierend verbunden ist oder unmittelbarer Teil des Verbindungskanals ist. Diese Druckkammer wird an einer Seite durch die reversibel verformbare elastische Membran verschlossen, welche hierdurch eine Veränderlichkeit des Innenvolumens der Druckkammer ermöglicht. Strömt Medium durch den Verbindungskanal, erhöht sich der Druck innerhalb der Druckkammer und die Membran wird ausgelenkt.

Der gemeinsam mit der Membran bewegte Verbindungsabschnitt ist elektrisch leitfähig und dadurch in der Lage, ein Schaltsignal zuzulassen, wenn er infolge der Auslenkung oder der Rückkehr aus dem ausgelenkten Zustand mit der Gegenkontaktfläche in Kontakt kommt oder sich von ihr löst. Anders als beim Ausführungsbeispiel der eingangs genannten DE 10 2013 214 601 B3 muss kein mit einem separaten Rückstellmittel wie einer Feder versehener Taster betätigt werden. Stattdessen wird der für den Schaltimpuls erforderliche Strom unmittelbar durch die Membran bzw. den mit der Membran mitbewegten Verbindungsabschnitt geleitet. Dies erhöht die Erfassungssicherheit, da bereits ein geringerer Druck zum zuverlässigen Erzeugen eines Impulses ausreichen kann.

Die genannte Erfassungseinrichtung ist vorzugsweise mit einer elektronischen Auswerteeinrichtung verbunden, die insbesondere als elektronisches Zählwerk ausgebildet sein kann. Diese Auswerteeinrichtung stellt zwei Signalleitungen zur Verfügung. Die Auswerteeinrichtung ist vorzugsweise dafür ausgebildet, ausgehend von einer elektrischen Trennung dieser beiden Signalleitungen eine zustande kommende Verbindung der Signalleitungen als Impuls zu interpretieren und in Reaktion hierauf einen Auswertevorgang, insbesondere einen Zählvorgang, durchzuführen. Statt dem Verbinden der zuvor getrennten Signalleitungen kann auch das Trennen der zuvor verbundenen Signalleitungen in dieser Art ausgewertet werden.

Eine besonders vorteilhafte Gestaltung der Erfassungseinrichtung sieht vor, dass diese zwei elektrische Gegenkontaktflächen aufweist, die mit jeweils einer der beiden Signalleitungen der elektrischen Auswerteeinrichtung verbunden sind. Diese Gegenkontaktflächen sind zueinander ortsfest und zunächst gegeneinander elektrisch getrennt ausgebildet. Sie sind derart angeordnet, dass sie durch Auslenkung des membranseitigen elektrisch leitfähigen Verbindungsabschnitts elektrisch miteinander verbunden werden können. Alternativ können sie auch durch die Rückkehr des elektrisch leitfähigen Verbindungsabschnitts aus dem ausgelenkten Zustand der Membran miteinander verbunden werden. Die Membran bzw. der hieran vorgesehene Verbindungsabschnitt agiert bei einer solchen Gestaltung als elektrische Brücke.

Bei einer hierzu alternativen Gestaltung ist die elektrische Gegenkontaktfläche mit einer Signalleitung verbunden, während die andere Signalleitung dauerhaft mit der Membran bzw. dem daran vorgesehenen Verbindungsabschnitt verbunden ist. Anders als bei der vorangegangenen Gestaltung, bei der der Verbindungsabschnitt bestimmungsgemäß mit beiden Gegenkontaktflächen in Kontakt kommt und dann als Brücke agiert, muss bei der letztgenannten Variante der membranseitige Verbindungsabschnitt durch die Auslenkung nur mit einer Gegenkontaktfläche in Kontakt gelangen. Mit der anderen Signalleitung ist der membranseitige Verbindungsabschnitt permanent gekoppelt, so dass ein Verarbeitungs- oder Zählimpuls bereits durch den Kontakt des membranseitigen Verbindungsabschnitts mit der nur einen Gegenkontaktfläche erfolgt.

Um die Membran in der beschriebenen Art und Weise zumindest abschnittsweise elektrisch leitfähig zu machen, sind mehrere Gestaltungsmöglichkeiten zweckmäßig. Gemäß einer ersten Variante ist die Membran mehrlagig ausgebildet und umfasst mindestens eine Kunststoffschicht und mindestens eine hiervon unterschiedliche elektrisch leitfähige Verbindungsschicht, die den leitfähigen Verbindungsabschnitt bildet. Durch die Zwei- oder Mehrlagigkeit kann die Membran alle gestellten Anforderungen besonders gut erfüllen. Die leitfähige Verbindungsschicht kann bei geringem Widerstand den gewünschten Kontakt bilden, während die vorzugsweise nicht leitfähige Kunststoffschicht im Hinblick auf die Verformungseigenschaften und Rückstelleigenschaften der Membran optimiert sein kann. Bei einer solchen zwei- oder mehrlagigen Membran erstreckt sich die Verbindungsschicht über die Gesamtfläche der Membran.

Bei einer zweiten Alternative, die nicht vom Gegenstand des Anspruchs 1 umfasst ist, ist die Membran lediglich in einem Kontaktbereich mit einem elektrisch leitfähigen Verbindungsabschnitt versehen, welcher beispielsweise in Art eines kleinen Metallplättchens vorliegen könnte, welches durch eine Klebeverbindung mit der Membran verbunden ist oder von dieser umspritzt ist.

Eine dritte Alternative sieht vor, dass die Membran mindestens eine Schicht aus einem elektrisch intrinsisch leitfähigen Kunststoff aufweist oder als Ganzes aus einem solchen gefertigt ist. Solche intrinsisch leitfähigen Kunststoffe bedürfen keines Zusatzes, um eine elektrische Verbindung zu schaffen. Allerdings weisen sie einen vergleichsweise hohen spezifischen Widerstand auf, so dass die elektronische Auswerteeinrichtung eine vergleichsweise hohe Empfindlichkeit aufweisen muss.

Statt eines intrinsisch leitfähigen Kunststoffs ist es auch möglich, einen als reinen Kunststoff nicht leitfähigen Kunststoff durch leitfähige Füllstoffe insgesamt leitfähig zu machen. Dies kann beispielsweise durch die Einlagerung von Graphit erfolgen.

Die Membran kann als separates Bauteil ausgebildet sein, welches in Randbereichen mit dem Gehäuse des Spenders verbunden wird, um eine vom Gehäuse nicht verschlossene Öffnung der Druckkammer zu verschließen. Besondere Vorteile im Hinblick auf die Dichtheit und die Herstellungskosten lassen sich erzielen, wenn nicht verformbare Abschnitte des Gehäuses, insbesondere nicht auslenkbare Wandungen der Druckkammer und/oder des Verbindungskanals einerseits und die Membran oder zumindest eine Schicht der Membran andererseits einstückig aus einem übereinstimmenden Kunststoff gefertigt sind. Die Auslenkbarkeit der Membran wird in diesem Falle durch die Dünnwandigkeit des Kunststoffs erzielt. Ebenfalls vorteilhaft kann es sein, das Gehäuse oder zumindest Teilabschnitte des Gehäuses einerseits und die Membran andererseits durch Mehrkomponentenspritzguss einstückig zu fertigen. Hierbei werden in einem zweistufigen Spritzgussvorgang mit veränderlicher Kavität Gehäuseabschnitte und die Membran aus unterschiedlichen Kunststoffmaterialien hergestellt. In einem Kontaktbereich schmilzt der zweite Kunststoff jedoch den ersten auf, so dass es zu einer besonders innigen und dichten Verbindung kommt. Der Mehrkomponentenspritzguss ist insbesondere dann von Vorteil, wenn die Membran zumindest teilweise aus einem intrinsisch leitfähigen Kunststoff oder aus einem durch Füllstoffe leitfähig gemachten Kunststoff besteht.

Membranen sind grundsätzlich am einfachsten herzustellen, indem diese extrudiert werden, woraus sich eine einheitliche Membrandicke ergibt. Besonders von Vorteil ist es jedoch, wenn die Membran durch Kunststoffspritzguss oder ein anderes Verfahren hergestellt wird, welches es gestattet, die Membrane mit einer unterschiedlichen Dicke zu versehen oder sie mit mindestens einer Sicke zu versehen. Durch ein Abweichen der Membran von der gleichbleibenden Dicke oder der rein ebenen Form lassen sich Versteifungen und/oder Bereiche erleichterter Verformbarkeit schaffen, welche zu einem definierteren Verformen der Membran beitragen können. Hierdurch wird es erleichtert, die Membran flächig mit der Gegenkontaktfläche oder den Gegenkontaktflächen in Kontakt zu bringen.

### KURZBESCHREIBUNG DER ZEICHNUNGEN

Weitere Vorteile und Aspekte der Erfindung ergeben sich aus den nachfolgend beschriebenen Ausführungsbeispielen, welche anhand der Figuren erläutert werden. Hierbei zeigen:
- Fig. 1: den Grundaufbau einer Ausführungsform eines erfindungsgemäßen Spenders in teilgeschnittener Ansicht,
- Fig. 2A und 2B: eine Erfassungseinrichtung zur Erfassung von Austragvorgängen vor und während eines Austragvorgangs,
- Fig. 3: zwei auf einer Platine angeordnete Gegenkontaktflächen zum Zusammenwirken mit einer Membran des Spenders der Fig. 1 bis 2B,
- Fig. 4A bis 4E: verschiedene Ausgestaltungen der Membran des Spenders der Fig. 1 bis 3 und
- Fig. 5A, 5B und 6: eine weitere Variante mit nur einer Gegenkontaktfläche, gegenüber derer die Membran beweglich ist.

### DETAILLIERTE BESCHREIBUNG DER AUSFÜHRUNGSBEISPIELE

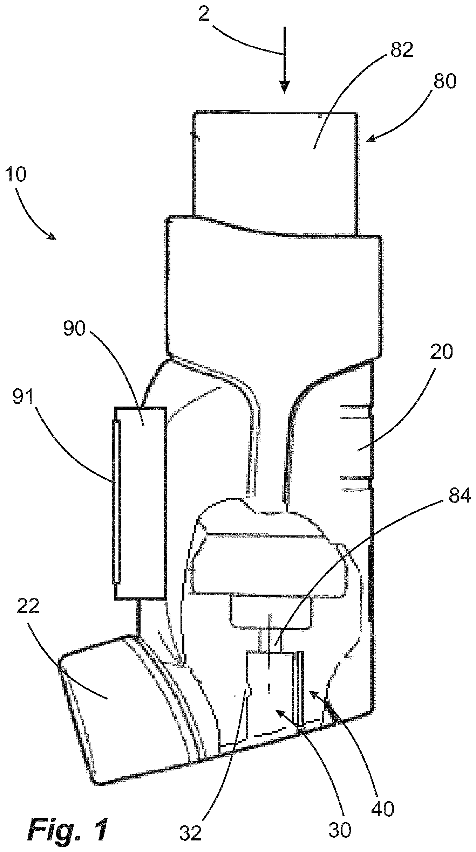

Fig. 1 zeigt in schematischer Ansicht einen erfindungsgemäßen Spender 10. Dieser ist als Inhalationsspender ausgebildet. Solche Spender werden üblicherweise als MDI oder PMDI bezeichnet.

Der Spender 10 verfügt über ein Gehäuse 20, in das eine Containereinheit 80 eingesetzt ist. Diese Containereinheit 80 ihrerseits verfügt über einen Mediumspeicher 82 sowie einen Auslassstutzen 84, der gegenüber dem Mediumspeicher 82 verlagerbar ist und im Zuge einer solchen Verlagerung eine abgemessene Mediummenge durch einen Austrittskanal im Auslassstutzen 84 abgibt. Die Containereinheit 80 ist von oben in das Gehäuse 20 eingeschoben, so dass der Auslassstutzen 84 in eine bodenseitige Aufnahme 30 hineinragt. Durch Niederdrücken des Mediumspeichers 82 in Richtung des Pfeils 2 kann somit ein Austragvorgang bewirkt werden, der dazu führt, dass Medium aus dem Mediumspeicher 82 durch eine Austragöffnung 32 der Aufnahme ausgegeben wird. Dies geschieht, während der Benutzer durch ein Mundstück 22 des Gehäuses 20 einatmet. Das durch die Austragöffnung 32 abgegebene Medium vermengt sich mit der durch den Benutzer eingesogenen Luft und gelangt so bestimmungsgemäß in die Lunge des Benutzers.

Der Spender 10 verfügt über eine Erfassungseinrichtung 40 und eine Auswerteeinrichtung 90, welche als elektrisches Zählwerk ausgebildet ist. Dieses Zählwerk 90 erhält von der Erfassungseinrichtung 40 einen Zählimpuls, wenn Medium aus dem Mediumspeicher 82 zur Austragöffnung 32 gefördert wird. Durch das Zählen der Austragvorgänge und das Darstellen des aktuellen Standes auf einem Display 91 ist es dem Benutzer möglich, die verbleibende Füllmenge im Mediumspeicher 82 abzuschätzen und somit zu vermeiden, unerwarteter Weise kein Medium mehr zur Verfügung zu haben.

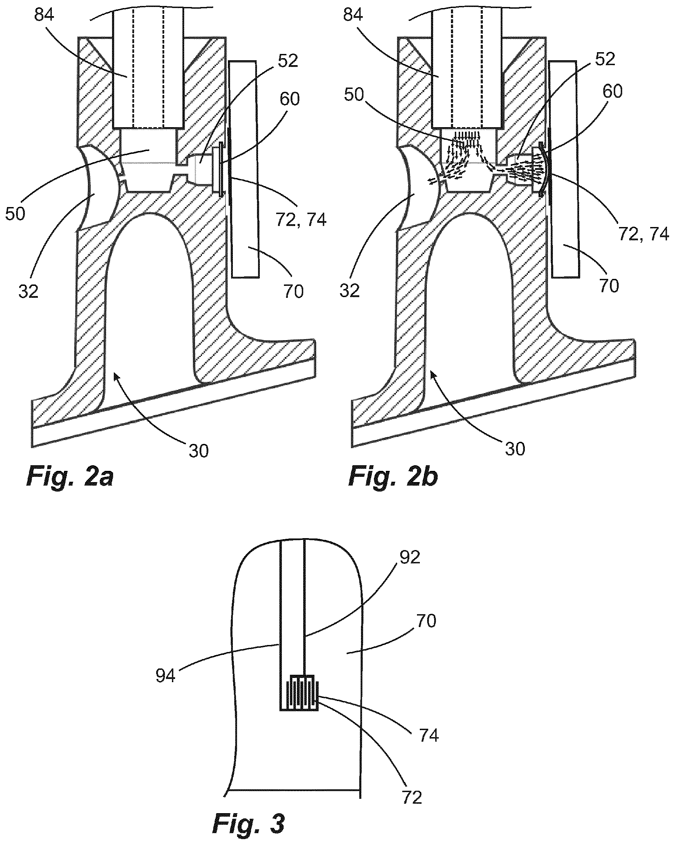

Die Fig. 2A und 2B zeigen die Erfassungseinrichtung 40 in vergrößerter Form. Wie den genannten Figuren zu entnehmen ist, ist innerhalb der Aufnahme 30 für den Auslassstutzen 84 ein Verbindungskanal 50 vorgesehen. Dieser verfügt über eine abgezweigte Druckkammer 52, in welcher der Druck steigt, wenn in der in Fig. 2B dargestellten Weise Medium aus dem Auslassstutzen 84 zur Austragöffnung 32 strömt.

Die Druckkammer 52 wird auf einer Seite durch eine Membran 60 begrenzt, welche in der in Fig. 2 dargestellten Weise verformt wird, wenn ein Überdruck in der Druckkammer 52 gegeben ist. Außenseitig der Druckkammer ist eine Platine 70 vorgesehen. Auf dieser in Fig. 3 nochmals dargestellten Platine 70 sind zwei Kontaktflächen 72, 74 angebracht, die mit jeweils einer Signalleitung 92, 94 verbunden sind, welche ihrerseits in nicht näher dargestellter Weise mit der Auswerteeinrichtung 90 gekoppelt sind. Die metallischen Kontaktflächen 72, 74 sind kammartig verschränkt angeordnet, so dass jeweils mehrere Teilabschnitte dieser Kontaktflächen unmittelbar zueinander benachbart sind. Dabei sind die Kontaktflächen jedoch voneinander galvanisch getrennt.

Die Membran 60 ist zumindest an ihrer in Richtung der Kontaktflächen 72, 74 weisenden Seite elektrisch leitfähig ausgebildet. Wenn die Membran in der in Fig. 2B verdeutlichten Art ausgelenkt wird, legt sie sich an die kammartig verschränkten Kontaktflächen 72, 74 an und verbindet diese. Hierdurch werden die Signalleitungen 92, 94 verbunden. Dies kann durch die Auswerteeinrichtung 90 erfasst werden und in Reaktion hierauf ein Zählschritt vorgenommen werden. Der aktualisierte Wert, der Auskunft über die bereits abgegebenen oder noch verbleibenden Dosen im Mediumspeicher 82 gibt, wird anschließend auf dem Display 91 dargestellt.

Dadurch, dass die Membran selbst den Kontakt schließt und ein mechanisches Einwirken auf weitere Komponenten zum Zwecke der Verlagerung derselben nicht vorgesehen ist, wird ein sehr zuverlässiges Zählen durch die Auswerteeinrichtung 90 möglich.

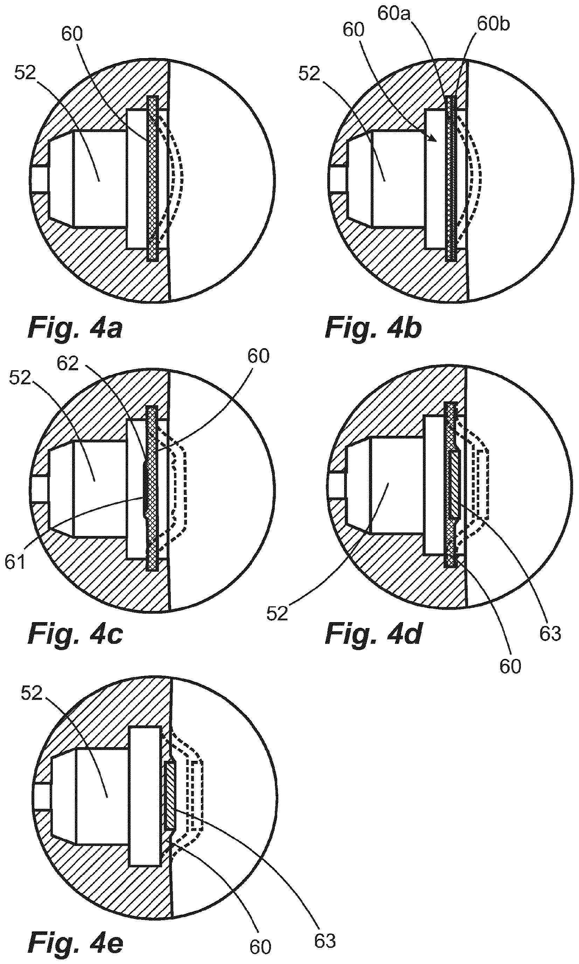

Die Fig. 4A bis 4E zeigen verschiedene Varianten der Druckkammer 52 und der die Druckkammer abschließenden Membran 60. Jeweils gestrichelt dargestellt ist die Form der Membran im ausgelenkten Zustand.

Bei der Gestaltung gemäß Fig. 4A findet eine als Ganzes leitfähige Membran 60 Verwendung, die vorzugsweise von Wandungsabschnitten des Gehäuses 20 umspritzt ist, welche aus einem nicht leitfähigen Kunststoff gefertigt sind. Die als Ganzes leitfähige Membran 60 der Fig. 4A kann durch Ausgestaltung aus einem intrinsisch leitfähigen Kunststoff erzielt werden. Auch ist es möglich, einen an sich nicht leitfähigen Kunststoff durch Additive/Füllstoffe leitfähig zu machen. Hierfür kommen beispielsweise eingelagerte Metallpartikel in Frage.

Bei der Variante gemäß Fig. 4B ist die Membran 60 zweilagig ausgebildet. Eine zur Druckkammer hin weisende Lage 60a der Membran besteht aus nicht leitfähigem Kunststoff und dient insbesondere als elastisch verformbarer Träger für die außenseitige Lage 60b, welche elektrisch leitfähig ist, beispielsweise da sie aus leitfähigem Metall besteht. Bei den beiden Lagen 60a, 60b kann es sich um isoliert hergestellte Lagen handeln, die zu einer Verbundmembran zusammengefügt wurden. Zweckmäßig kann es auch sein, eine solche Membran dadurch herzustellen, dass die leitende Lage 60b auf der Trägerlage 60a abgeschieden wird, beispielsweise durch Bedampfen.

Bei der Ausgestaltung der Fig. 4C ist wiederum eine als Ganzes leitfähige Membran 60 vorgesehen, deren Besonderheit jedoch darin liegt, dass ein zentrischer Bereich 61 dieser Membran durch eine ringförmige Verdickung 62 umgeben ist. Dies führt zu einer Versteifung, die auf die Form der Membran 60 im ausgelenkten Zustand Einfluss nimmt. Wie der Fig. 4C zu entnehmen ist, wird der zentrische Bereich 61 durch eine solche Versteifung weitgehend unverformt ausgelenkt. Dies erleichtert es, zuverlässig erfassbaren flächigen Kontakt der Membran 60 mit den beiden Kontaktflächen 72, 74 zu schaffen.

Die nicht erfindungsgemäße Ausgestaltung der Fig. 4D weist ähnlich jener der Fig. 4B eine Membran mit Abschnitten aus verschiedenen Materialien auf. Allerdings ist hier der leitfähige Bereich der Membran nicht durch eine die gesamte Membran überdeckende Membranlage geschaffen, sondern durch einen nur zentrisch an der Membran 60 vorgesehenen Verbindungsabschnitt 63, der beispielsweise in Art eines kleinen Metallplättchens ausgebildet sein kann. Dieses Metallplättchen 63 kann mit einem elastisch auslenkbaren Abschnitt der Membran verklebt sein oder stattdessen in diesem eingebettet sein. Auch hierdurch wird eine vorteilhafte Versteifung erzielt.

Fig. 4E ist eine ebenfalls nicht erfindungsgemäße Variante zu der Gestaltung der Fig. 4D. Auch hier findet ein Metallplättchen 63 Anwendung. Die Besonderheit bei der Ausgestaltung gemäß Fig. 4E liegt darin, dass die Membran 60 im Übrigen einstückig mit den umgebenden Abschnitten des Gehäuses 20 ausgebildet ist und aus identischem Material besteht. Nur durch die Dünnwandigkeit der Membran wird die gewünschte Auslenkbarkeit erzielt.

Neben den dargestellten Varianten ist auch eine Vielzahl von Kombinationen denkbar.

Weiterhin ist neben der Einstückigkeit der Membran bzw. von Abschnitten der Membran mit dem umgebenden Gehäuse gemäß Fig. 4E auch möglich, statt der dort verwendeten identischen Materialien für die Membran und das umgebende Gehäuse unterschiedliche Materialien zu verwenden, die im Zweikomponentenspritzguss stoffschlüssig und innig zusammengefügt werden. Dies bietet sich insbesondere bei Nutzung von elektrisch leitfähigen Kunststoffen für die Membran an, wie es im Falle der Fig. 4A und 4C vorgesehen ist.

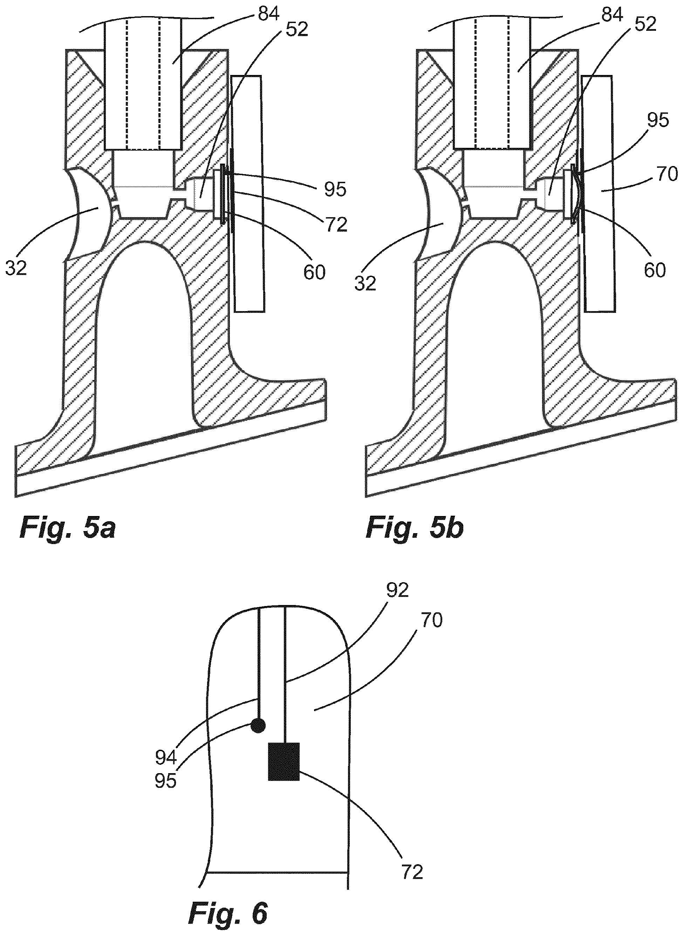

Die Fig. 5A bis 6 zeigen eine alternative Gestaltung. Bei dieser alternativen Gestaltung ist auf der Leiterplatte 70 nur eine Kontaktfläche 72 vorgesehen, welche mit der Signalleitung 92 verbunden ist. Die andere Signalleitung 94 ist dagegen über einen kurzen Verbindungsabschnitt 95 dauerhaft mit der Membran 60 verbunden, welche in gleicher Weise, wie in den Fig. 4A bis 4E gezeigt, ausgebildet sein kann. Der Kontakt, welcher durch die Auswerteeinrichtung 90 zum Zählen der Austragvorgänge erfasst wird, wird somit unmittelbar zwischen der Membran 60 einerseits und der Kontaktfläche 72 geschaffen. Hierdurch wird es möglich, die Kontaktfläche 72 vergleichsweise groß und ununterbrochen auszugestalten, so dass eine nochmals erhöhte Zuverlässigkeit in Hinblick auf die durch Membranverformung erzielte galvanische Kopplung der Signalleitungen 92, 94 erzielt wird.

## Patentansprüche

1. Pharmazeutischer Spender (10) mit
- einem Gehäuse (20),
- einem Mediumspeicher (82),
- einer Austragöffnung (32),
- einem Verbindungskanal (50), der den Mediumspeicher (82) mit der Austragöffnung (32) verbindet und
- einer Erfassungseinrichtung (40), die im Bereich des Verbindungskanals (50) angeordnet ist und zur Erfassung des im Verbindungskanal (50) strömenden Mediums ausgebildet ist,
wobei
- der Verbindungskanal (50) mit einer Druckkammer (52) kommunizierend verbunden ist,
- die Erfassungseinrichtung (40) eine Membran (60) umfasst, die
o die Druckkammer (52) zumindest abschnittsweise begrenzt,
o einen elektrischen leitfähigen Verbindungsabschnitt (60, 60b) umfasst und
o durch einen Druckanstieg in der Druckkammer (52) druckbeaufschlagbar und dadurch gemeinsam mit dem leitfähigen Verbindungsabschnitt (60, 60b) auslenkbar ist,
- die Erfassungseinrichtung (40) mindestens eine elektrische Gegenkontaktfläche (72, 74) aufweist, gegenüber derer der membranseitige Verbindungsabschnitt (60, 60b) durch Druckbeaufschlagung verlagerbar ist, so dass hierdurch ein elektrischer Kontakt zwischen dem leitfähigen Verbindungsabschnitt (60, 60b) und der Gegenkontaktfläche (72, 74) geschaffen oder getrennt wird,
**dadurch gekennzeichnet, dass**
- die Membran (60) mehrlagig ausgebildet ist und mindestens umfasst:
o eine Kunststoffschicht (60a) und
o eine elektrisch leitfähige Verbindungsschicht (60b), die den leitfähigen Verbindungsabschnitt (60b) bildet, oder
- die Membran (60) mindestens eine Schicht aus einem elektrisch intrinsisch leitfähigen Kunststoff aufweist oder
- die Membran (60) zumindest abschnittsweise aus einem Kunststoff besteht, der mit mindestens einem leitfähigen Füllstoff versehen ist, vorzugsweise mit Graphit.

2. Pharmazeutischer Spender (10) nach Anspruch 1,
**gekennzeichnet durch**
eine elektronische Auswerteeinrichtung (40), insbesondere in Form eines elektronischen Zählwerks, die über zwei Signalleitungen (92, 94) verfügt, deren Verbindung oder deren Trennung von der Auswerteeinrichtung einen Auswertevorgang, insbesondere einen Zählvorgang, ausübt.

3. Pharmazeutischer Spender (10) nach Anspruch 2,
**dadurch gekennzeichnet, dass**
die Erfassungseinrichtung (40) zwei elektrische Gegenkontaktfläche (72, 74) aufweist, die
- mit den beiden Signalleitungen (92, 94) der elektrischen Auswerteeinrichtung (40) verbunden sind,
- zueinander ortsfest angeordnet sind und
- durch die Auslenkung des membranseitigen elektrisch leitfähigen Verbindungsabschnitts (60, 60b) miteinander verbindbar oder voneinander trennbar sind.

4. Pharmazeutischer Spender (10) nach Anspruch 2,
**dadurch gekennzeichnet, dass**
- die elektrische Gegenkontaktfläche (72) mit einer der Signalleitungen (92) verbunden ist und
- der membranseitige Verbindungsabschnitt (60, 60b) dauerhaft mit der anderen Signalleitung (94) verbunden ist.

5. Pharmazeutischer Spender (10) nach einem der vorstehenden Ansprüche,
**dadurch gekennzeichnet, dass**
die Membran (60) und umgebende bestimmungsgemäß nicht auslenkbare Wandungen der Druckkammer (52) und/oder des Verbindungskanals (50)
- einstückig und aus übereinstimmendem Kunststoff gefertigt sind oder
- einstückig und durch Mehrkomponentenspritzguss gefertigt sind.

6. Pharmazeutischer Spender (10) nach einem der vorstehenden Ansprüche,
**dadurch gekennzeichnet, dass**
die Membran (60) Bereiche verschiedener Dicke aufweist oder die Membran (60) mit mindestens einer Sicke oder abschnittsweisen Verdickung (62) versehen ist.

7. Pharmazeutischer Spender (10) nach einem der vorstehenden Ansprüche,
**dadurch gekennzeichnet, dass**
der Spender (10) als Inhalationsspender ausgebildet ist, der ein Gehäuse mit einer Aufnahme zum Einsetzen einer wechselbaren Containereinheit (80) ausgebildet ist, die den Mediumspeicher (82) umfasst.

## Claims

1. Pharmaceutical dispenser (10) with
- a housing (20),
- a medium reservoir (82),
- a discharge opening (32),
- a connecting duct (50) which connects the medium reservoir (82) to the discharge opening (32) and
- a detection device (40) which is arranged in the region of the connecting duct (50) and is designed to detect the medium flowing in the connecting duct (50),
wherein
- the connecting duct (50) is connected in communicating manner to a pressure chamber (52),
- the detection device (40) includes a membrane (60) which
∘ bounds the pressure chamber (52) at least intermittently,
∘ includes an electrically conductive connecting portion (60, 60b) and
∘ is capable of being pressurized by a rise in pressure in the pressure chamber (52) and is thereby capable of being deflected together with the conductive connecting portion (60, 60b),
- the detection device (40) exhibits at least one electrical mating contact surface (72, 74), with respect to which the membrane-side connecting portion (60, 60b) is capable of being displaced by application of pressure, so that by this means an electrical contact between the conductive connecting portion (60, 60b) and the mating contact surface (72, 74) is created or broken, **characterized in that**
- the membrane (60) is formed in multiple layers and comprises at least:
∘ a plastic layer (60a) and
∘ an electrically conductive connecting layer (60b) which forms the conductive connecting portion (60b), or
- the membrane (60) exhibits at least one layer of an electrically intrinsically conductive plastic or
- the membrane (60) consists at least intermittently of a plastic that is provided with at least one conductive filler, preferentially with graphite.

2. Pharmaceutical dispenser (10) according to Claim 1, **characterized by**
an electric evaluating device (40), in particular in the form of an electronic counter, which is provided with two signal lines (92, 94), the connection of which or the disconnection of which from the evaluating device performs an evaluating process, in particular a counting process.

3. Pharmaceutical dispenser (10) according to Claim 2, **characterized in that**
the detection device (40) exhibits two electrical mating contact surfaces (72, 74) which
- are connected to the two signal lines (92, 94) of the electronic evaluating device (40),
- are arranged to be fixed in relation to one another and
- are capable of being connected to one another or disconnected from one another by the deflection of the membrane-side electrically conductive connecting portion (60, 60b).

4. Pharmaceutical dispenser (10) according to Claim 2, **characterized in that**
- the electrical mating contact surface (72) is connected to one of the signal lines (92) and
- the membrane-side connecting portion (60, 60b) is permanently connected to the other signal line (94).

5. Pharmaceutical dispenser (10) according to one of the preceding claims,
**characterized in that**
the membrane (60) and surrounding walls of the pressure chamber (52) and/or of the connecting duct (50), which are non-deflectable as intended
- have been manufactured in one piece and from matching plastic or
- have been manufactured in one piece and by multi-component injection molding.

6. Pharmaceutical dispenser (10) according to one of the preceding claims,
**characterized in that**
the membrane (60) exhibits regions of different thickness, or the membrane (60) is provided with at least one bead or intermittent thickening (62).

7. Pharmaceutical dispenser (10) according to one of the preceding claims,
**characterized in that**
the dispenser (10) takes the form of an inhalation dispenser which is designed as a housing with a receptacle for inserting an interchangeable container unit (80) which contains the medium reservoir (82).

## Revendications

1. Distributeur de produits pharmaceutiques (10) comprenant
- un boîtier (20),
- un réservoir de fluide (82),
- un orifice de sortie (32),
- un conduit de liaison (50) qui relie le réservoir de fluide (82) à l'orifice de sortie (32) et
- un moyen de détection (40) qui est disposé dans la région du canal de liaison (50) et qui est adapté pour détecter le fluide circulant dans le conduit de liaison (50),
dans lequel
- le conduit de liaison (50) communique avec une chambre de pression (52),
- le moyen de détection (40) comprend une membrane (60)
o qui délimite au moins partiellement la chambre de pression (52),
o qui comprend une partie de liaison électriquement conductrice (60, 60b), et
o qui peut être soumise à une pression par augmentation de la pression dans la chambre de pression (52) et qui peut ainsi être déviée en association avec la partie de liaison conductrice (60, 60b),
- le moyen de détection (40) comporte au moins une surface de contact électrique homologue (72, 74), contre laquelle la partie de liaison (60, 60b) côté membrane peut être déplacée, lorsqu'elle est soumise à une pression, de manière à établir ou supprimer ainsi un contact électrique entre la partie de liaison conductrice (60, 60b) et la surface de contact homologue (72, 74),
**caractérisé en ce que**
- la membrane (60) est réalisée de manière à comporter plusieurs couches et comprend au moins :
- une couche de matière plastique (60a) et
- une couche de liaison électriquement conductrice (60b) qui forme la partie de liaison conductrice (60b), ou
- la membrane (60) comporte au moins une couche constituée d'une matière plastique intrinsèquement électriquement conductrice ou
- la membrane (60) est constituée au moins partiellement d'une matière plastique qui est dotée d'au moins un agent de charge conducteur, de préférence de graphite.

2. Distributeur de produits pharmaceutiques (10) selon la revendication 1,
**caractérisé par**
un moyen d'évaluation électrique (40), notamment sous la forme d'un compteur électronique, qui comporte deux ligne de signaux (92, 94) dont la connexion au dispositif d'évaluation ou la déconnexion de celui-ci, déclenche un processus d'évaluation, notamment un processus de comptage.

3. Distributeur de produits pharmaceutiques (10) selon la revendication 2, **caractérisé en ce que** le moyen de détection (40) comporte deux surfaces de contact électriques homologues (72, 74)
- qui sont reliées aux deux lignes de signaux (92, 94) du moyen d'évaluation électronique (40),
- qui sont disposées en position fixe l'une par rapport à l'autre, et
- qui peuvent être connectées l'une à l'autre ou déconnectées l'une de l'autre par déviation de la partie de liaison éclectiquement conductrice (60, 60b) côté membrane.

4. Distributeur de produits pharmaceutiques (10) selon la revendication 2,
**caractérisé en ce que**
- la surface de contact électrique homologue (72) est reliée à l'une des lignes de signaux (92), et
- la partie de liaison (60, 60b) côté membrane est reliée en permanence à l'autre ligne de signal (94).

5. Distributeur de produits pharmaceutiques (10) selon l'une quelconque des revendications précédentes, **caractérisé en ce que**
la membrane (60) et des parois qui l'entourent ne pouvant normalement pas être déviées de la chambre de pression (52) et/ou du conduit de liaison (50)
- sont réalisées d'un seul tenant et sont constituées d'une matière plastique correspondante ou
- sont réalisées d'un seul tenant et sont formées par moulage par injection de composants multiples.

6. Distributeur de produits pharmaceutiques (10) selon l'une quelconque des revendications précédentes, **caractérisé en ce que**
la membrane (60) présente des zones d'épaisseurs différentes ou **en ce que** la membrane (60) est dotée d'au moins une ondulation ou d'un épaississement localisé (62).

7. Distributeur de produits pharmaceutiques (10) selon l'une quelconque des revendications précédentes, **caractérisé en ce que**
le distributeur (10) est réalisé sous la forme d'un distributeur à inhalation qui est réalisé sous la forme d'un boîtier comportant un logement permettant d'insérer un récipient interchangeable (80) comprenant un réservoir de fluide (82).
